# EUROPÄISCHE PATENTSCHRIFT

(11) **EP 0 968 725 B1**
(45) Veröffentlichungstag und Bekanntmachung des Hinweises auf die Patenterteilung: **07.06.2006**
(21) Anmeldenummer: 99890199.5
(22) Anmeldetag: 18.06.1999
(51) Int. Cl.: A61L 9/16, B09B 1/00

(54) **Verfahren und Vorrichtung zum Entkeimen der Abluft von Müllagerstätten und/oder Sortieranlagen**
Method and device for sterilising waste air from waste dumps and/or sorting plants
Procédé et dispositif pour stériliser de l'air d'échappement des décharges d'ordures et/ou des installations de tri

(30) Priorität: 30.06.1998 AT 113798
(43) Veröffentlichungstag der Anmeldung: 05.01.2000
(73) Patentinhaber: Knoch, Kern & Co., 9020 Klagenfurt (AT)
(72) Erfinder: Willitsch, Friedrich-Dipl.-Ing., 9585 Gödersdorf (AT)
(74) Vertreter: Hehenberger, Reinhard

(56) Entgegenhaltungen:
- DE-C- 3 705 364
- FR-A- 2 629 179
- US-A- 5 156 826

## Beschreibung

Die Erfindung betrifft ein Verfahren zum Vernichten von Keimen und anderen gesundheitsschädlichen Inhaltsstoffen in der Abluft von Müllagerstätten und/oder -sortieranlagen, bei dem die Abluft erhitzt wird. Die Erfindung betrifft desweiteren eine Vorrichtung zum Vernichten von Keimen und anderen gesundheitsschädlichen Inhaltsstoffen in der Abluft von Müllagerstätten und/oder -sortieranlagen mit wenigstens einer Einrichtung zum Lagern und/oder Sortieren von Müll.

Unter Müll wird im vorliegenden Fall der in privaten Haushalten sowie gewerblichen Betrieben anfallende Abfall (Hausmüll, hausmüllähnlicher Abfall aus gewerblichen Betrieben, auch Restmüll oder Systemmüll genannt) verstanden. Dieser zur Entsorgung bestimmte Müll enthält Komponenten, wie z.B. Kunststoffe, Textilien, Papier und Pappe, sowie Verbundmaterialien, welche aufgrund ihres Energieinhaltes (Heizwert) als Brennstoff eingesetzt werden können. Der Anteil an diesen Fraktionen kann in Summe bis 40% betragen. Einen nicht unerheblichen Anteil an der Masse des Mülls hat auch dessen Feuchtigkeit, die ebenfalls einen Anteil bis zu 40 Masse-% erreichen kann.

Damit die brennbaren Bestandteile des Mülls als Brennstoff verwendet werden können, muß dieser getrocknet werden. Beim Lagern und Trennen und insbesondere beim Trocknen des Mülls werden Keime und andere gesundheitsschädliche Inhaltsstoffe freigesetzt, die nicht ohne weiteres an die Umgebung abgegeben werden dürfen.

Die US-A-5,156,826 beschreibt ein Verfahren zum Beseitigen von Geruchsstoffen in Gasen und Dämpfen, die bei der Lebensmittel-, insbesondere bei der Bierherstellung, auftreten, wobei die organischen Geruchsstoffe in Anwesenheit von Luftsauerstoff bei höheren Temperaturen oxidieren.

Aus der DE-C-37 05 364 ist es bekannt, Abluft von krankenhausspezifischen Abfällen in einer Hochtemperatur - Desinfektionsanlage zu stützen, sodass in Folge keine mit gesundheitsschädlichen Inhaltsstoffen beladene Abluft aus der Anlage austreten kann. Nachteilig bei dieser Anlage ist jedoch der hohe Energieverbrauch, der ausschließlich zum Erhitzen der Abluft benötigt wird.

Der vorliegenden Erfindung liegt daher die Aufgabe zugrunde, eine ökonomische Möglichkeit für eine Behandlung dieser Abluft zur Verfügung zu stellen, bei der die Keime oder anderen gesundheitsschädliche Inhaltsstoffe so weit wie möglich unschädlich gemacht werden.

Gelöst wird diese Aufgabe durch ein gattungsgemäßes Verfahren, das dadurch gekennzeichnet ist, daß die Abluft einem Teil einer Wärme erzeugenden bzw. abgebenden Industrieanlage zugeführt wird und daß die Abluft auf eine die Keime und anderen gesundheitsschädlichen Inhaltsstoffe abtötende Mindesttemperatur erhitzt wird.

Diese Aufgabe wird weiters bei einer gattungsgemäßen Vorrichtung dadurch gelöst, daß die Einrichtung über eine Verbindungsleitung mit einem Wärme abgebenden bzw. erzeugenden Teil einer Industrieanalage verbunden ist, in der die Abluft auf eine die Keime und anderen gesundheitsschädlichen Inhaltsstoffe abtötende Mindesttemperatur erhitzt wird.

Bei Industrieanlagen werden häufig Kühler eingesetzt, die mit Betriebstemperaturen in einer Höhe arbeiten, bei der eine zuverlässige Abtötung der Keime und anderen gesundheitsschädlichen Inhaltsstoffe der Müllagerstätte bzw. -sortieranlage gewährleistet ist. Beispielhaft wird auf Anlagen zur Zementklinkerherstellung verwiesen, in welchen das zu Rohmehl vermahlene Gestein zuerst bei Temperaturen um 1000°C vorkalziniert und dann in einem Drehrohr bei Guttemperaturen von ca. 1400 bis 1500°C und Flammtemperaturen über 2000°C gesintert wird. Um die beim Sintern entstandenen Klinkerphasen entsprechend zu erhalten, muß der Klinker nach dem Verlassen des Drehrohres abgekühlt werden. Dies geschieht in einem sogenannten Klinkerkühler.

Klinkerkühler können als Planeten-Rohr-Schacht- oder Rostkühler ausgebildet sein. Die am häufigsten eingesetzte Klinkerkühlerbauart ist der Rostkühler, bei dem die erzielten Klinkerendtemperaturen zwischen 120 und 300°C liegen. Die durchschnittliche Eintrittstemperatur des Klinkers in den Kühler beträgt ca. 1000 bis 1100°C. Der Kühler ist unterhalb des Rostes in Kammern unterteilt. Je nach Erzeugungsmenge (Ofengröße und Kühlerauslegung) sind unterschiedlich viele Kühlkammern vorhanden. Die Kühlung erfolgt erfindungsgemäß durch die Abluft der Müllagerstätte bzw. -sortieranlage, die mittels Ventilatoren in die einzelnen Kammern gepreßt und durch das heiße Klinkerbett gedrückt wird.

Für die vollständige Verbrennung des für die Beheizung des in den Drehrohrofen eingebrachten Brennstoffes wird der Großteil der Verbrennungsluftmenge aus dem Kühler entnommen. Diese Luftmenge wird als sogenannte Sekundärluft bezeichnet. Bei Planeten-, Rohr- oder Schachtkühlern wird die gesamte Kühlluft als Sekundärluft verwendet, beim Rostkühler nur die für die Verbrennung unbedingt notwendige Luftmenge.

Bei Rostkühlern verläßt ein großer Teil der Kühlerabluft den Kühler direkt und wird nach entsprechender Filterung über einen Abluftkamin an die Umgebung abgegeben. Die durchschnittliche Ablufttemperatur liegt über 200°C. Verschiedene Möglichkeiten der Wärmerückgewinnung können noch zwischengeschaltet sein.

Es ist ersichtlich, daß bei der erfindungsgemäßen Vorrichtung die Abluft aus der Müllagerstätte bzw. -sortieranlage in einen Temperaturbereich erwärmt wird, bei dem eine zuverlässige Abtötung von Keimen und anderen gesundheitsschädlichen Inhaltsstoffen durch Hitze gewährleistet ist, wobei es sich um eine sehr ökonomische Form der Sterilisierung der Abluft handelt, da für den Kühler ohnedies große Mengen an Kühlluft erforderlich sind.

Alternativ kann die Abluft auch direkt einem Brenner oder Ofen einer beliebigen Anlage, z.B. eines Fernheizwerkes, als Verbrennungsluft zugeführt werden, da die Abluft auch brennbare Bestandteile aufweisen kann.

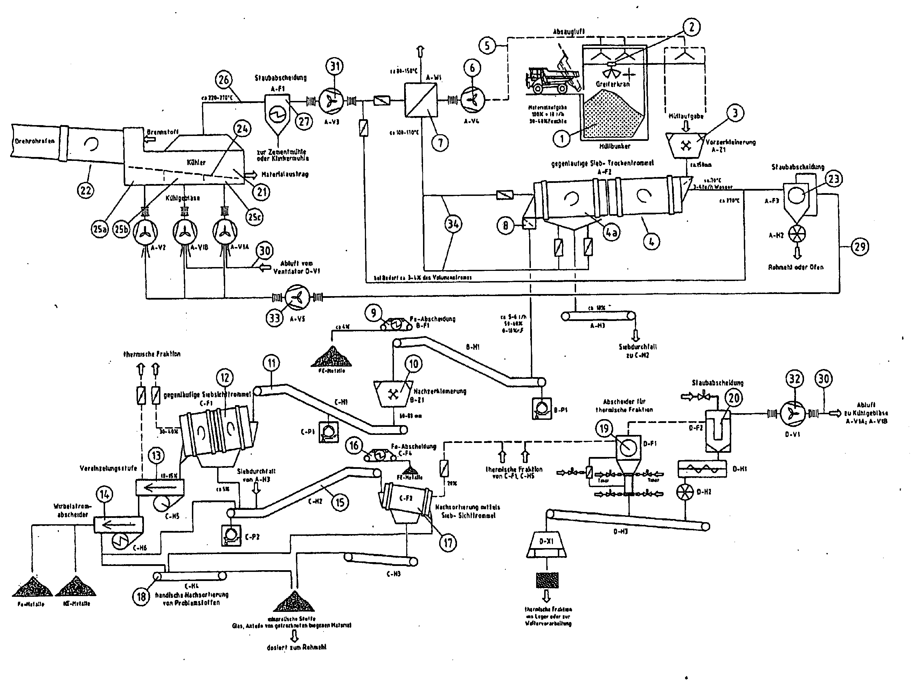

Weitere Merkmale und Vorteile der Erfindung ergeben sich aus den Unteransprüchen und der nachfolgenden Beschreibung eines bevorzugten Ausführungsbeispieles der Erfindung mit Bezug auf die beiliegende Zeichnung.

In der Zeichnung ist mit 1 eine als Müllbunker bezeichnete Müllagerstätte dargestellt, die z.B. mittels Lastwagen oder Bahn mit Müll beschickt wird. Die Größe des Müllbunkers 1 ist abhängig vom Müllaufkommen der Kapazität und der Betriebsweise der nachgeschalteten Sortieranlage. Aus dem Müllbunker 1 wird der Müll mittels eines Greiferkranes 2 auf ein Vorzerkleinerungsaggregat 3 aufgegeben. Die Zerkleinerungsgröße in diesem Aggregat 3 beträgt ca. 150 bis maximal 200 mm. Nach diesem Aggregat 3 gelangt der Müll in eine Siebtrockentrommel 4. Die Feuchtigkeit des Mülls vor der Siebtrockentrommel 4 kann bis 40 Gew.-% und mehr betragen. Die Siebtrockentrommel 4 ist in zwei Abschnitte 4a, 4b geteilt, die in entgegengesetzte Richtungen gedreht werden, um eine Entwirrung des Mülls zu erreichen und damit eine bessere Trocknung zu gewährleisten. In der Siebtrockentrommel 4 wird der Müll auf eine Restfeuchte von ca. 10% getrocknet. Die Abluft aus dem Müllbunker 1 und dem Zerkleinerungsaggregat 3 wird über eine Leitung und einen Ventilator 6 abgesaugt und über einen Wärmetauscher 7, in dem die Abluft auf ca. 130 bis 170°C erwärmt wird, der Siebtrockentrommel 4 über eine Leitung 34 zugeführt, um den vorzerkleinerten Müll zu trocknen. Dabei werden gleichzeitig verschiedene, im Müll befindliche Parasiten unschädlich gemacht.

Je nach Ausbildung des Abschnittes bzw. Siebteils 4a kann die Korngröße des Siebdurchfalls bestimmt werden. Die Siebtrockentrommel 4 ist weiters so ausgeführt, daß die Siebung erst nach der Trocknung durchgeführt wird. Über ein besonderes unter Unterdruck betriebenes Austragssystem 8 verläßt der Müll die Trommel 4.

Nach der Siebtrockentrommel 4 werden eisenhältige Materialien über einen Magnetabscheider 9 abgeschieden. Nach der Abscheidung von Eisen und Metall folgt ein weiteres Zerkleinerungsaggregat 10 mit einer Zerkleinerungsgröße auf ca. 60 bis 80 mm. Anschließend wird der Müll über eine Förderstrecke 11 einer weiteren, ebenfalls gegenläufig ausgebildeten Siebsichttrommel 12 zugeführt. In dieser Siebsichttrommel 12 wird die energiereiche Fraktion des Mülls, wie Kunststoffe, Papier, Verbundmaterialien, Textilien, usw., abgeschieden.

Dieser Anteil der heizwertreichen Fraktion im Müll beträgt ca. 30 bis 40%. Nachdem die Müllmenge bereits in der Siebtrockentrommel 4 um etwa 30 Gew.-% durch Trocknung reduziert wurde, werden hier nochmals etwa 20 bis 30 Gew.-% entfernt. Der aus der Siebsichttrommel 12 austretende, verbleibende Rest des Mülls wird über eine Vereinzelungsstrecke 13 einem Wirbelstromabscheider 14 zugeführt. Hier werden nochmals Eisen und Nichteisenmetalle abgetrennt.

Der Siebdurchfall der Siebtrockentrommel 4 und der Siebsichttrommel 12 werden dann zu einer Förderstrecke 15 zusammengeführt. Die Zusammensetzung dieses Siebdurchfalls stellt sich hauptsächlich wie folgt dar: mineralische Stoffe, Glas, Anteile von trockenem, biogenem Material, sowie ein geringer Anteil von Problemstoffen, kleineren Anteilen an Eisen und Nichteisenmetall, sowie einem Anteil von kleinstückiger, thermischer Fraktion. Dieses Gemenge wird nach nochmaliger Eisenmetallabscheidung in einem Abscheider 16 einer weiteren als Siebsichttrommel ausgeführten Nachsortiertrommel 17 zugeführt. In dieser erfolgt eine Absichtung der restlichen thermischen Fraktion. Die Siebgröße wird so gewählt, daß eventuell darin vorhandene Problemstoffe, wie z.B. Batterien, die Trommel im geraden Durchlauf verlassen.

Die vom Wirbelstromabscheider 14 übriggebliebene Fraktion, sowie der Durchlauf der Nachsortiertrommel 17 werden zusammengeführt und einer Einheit 18 zur visuellen Kontrolle und zum händischen Nachsortieren zum Ausscheiden der Problemstoffe zugeführt. Die vom Wirbelstromabscheider 14 übriggebliebene Fraktion kann bei Bedarf ebenfalls zur Nachsortiertrommel 17 geführt werden. Der Siebdurchfall aus der Nachsortiertrommel 17, sowie das übriggebliebene Gemenge aus der händischen Nachsortierung bleibt mit einem Anteil von 5 bis 10% der ursprünglichen Müllmenge als unverwertbarer Rest zurück.

Die thermische Fraktion aus der Siebsichttrommel 12 sowie aus der Nachsortiertrommel 17 wird mittels Luft abgesaugt, und einem Abscheidezyklon 19 zugeführt. Die Luft aus dem Abscheidezyklon 19 wird anschließend in einen Staubfilter 20 gefiltert und über ein Gebläse 32 in einer Leitung 30 einem Kühler 21 einer Anlage zur Zementklinkerherstellung 22 zugeführt. Desgleichen wird die Abluft aus der Siebtrockentrommel 4 einem Staubabscheider 23 zugeführt, und ebenfalls über ein Gebläse 33 durch eine Leitung 29 in den Kühler 21 eingeleitet.

Die Führung der gesamten Abluft aus dem Müllbunker 1, der Vorzerkleinerung 3, der Siebtrockentrommel 4, der Siebsichttrommel 12, der Nachsortiertrommel 17, sowie den Staubabscheidern 20 und 23 und den vorgeschalteten Einheiten erfolgt durch Sauggebläse 6, 32, 33, so daß diese Stellen immer unter Unterdruck gehalten werden, damit keine Luft nach außen dringen kann.

Der Kühler 21 ist im dargestellten Ausführungsbeispiel als Rostkühler ausgeführt, wobei unter dem Rost 24 z.B. drei Kammern 25a, 25b und 25c vorgesehen sind. Die Luft aus der Siebtrockentrommel 4 wird dabei allen drei Kammern 25a, 25b und 25c zugeführt, wogegen die Abluft aus dem Abscheidezyklon 19 bzw. dem Staubabscheider 20 nur den Kammern 25b, 25c zugeführt wird. Es ist aber möglich, die Verteilung der Zufuhr der Abluft aus den einzelnen Abschnitten der Sortieranlage den Kammern 25a, 25b und 25c auch anders aufgeteilt zuzuführen, was sich u.a. nach der jeweils anfallenden Abluftmenge, der Belastung mit Keimen und anderen gesundheitsschädlichen Inhaltsstoffen, sowie der Anzahl der Kammern richten kann.

Beim Durchtritt der Abluft durch das heiße Klinkerbett im Kühler 21 werden die gesundheitsschädlichen Stoffe zerstört, die Luft teilweise als Sekundärluft für den Brennvorgang verwendet und der Rest als Kühlerabluft abgeführt. Die Kühlerabluft wird von einem Gebläse 31 über eine Leitung 26 abgesaugt, durch einen Staubabscheider 27, darauf durch den Wärmetauscher 7 geführt und anschließend an die Umgebung abgegeben, oder einer weiteren thermischen Nutzung, z.B. für Heizzwecke, zugeführt.

Die aus der Siebsichttrommel 12 und der Nachsortiertrommel 17 abgesichtete, getrocknete, thermische Fraktion wird, wie bereits erwähnt, im Abscheidezyklon 19 abgeschieden und zur Weiterverarbeitung zwischengelagert oder direkt zum Einsatz als Sekundärbrennstoff weitergeleitet.

## Patentansprüche

1. Verfahren zum Abtöten von Keimen und anderen gesundheitsschädlichen Inhaltsstoffen in der Abluft von Müllagerstätten und/oder -sortieranlagen, bei dem die Abluft erhitzt wird,
**dadurch gekennzeichnet, daß** die Abluft einem Teil einer Wärme erzeugenden bzw. abgebenden Industrieanlage zugeführt wird und daß die Abluft auf eine die Keime und anderen gesundheitsschädlichen Inhaltsstoffe abtötende Mindesttemperatur erhitzt wird.

2. Verfahren nach Anspruch 1, **dadurch gekennzeichnet, daß** die Abluft durch einen Wärmetauscher und eine Trockentrommel für den Müll der Industrieanlage zugeführt wird.

3. Verfahren nach Anspruch 2, **dadurch gekennzeichnet, daß** der Wärmetauscher mit der Abwärme der Industrieanlage beheizt wird.

4. Verfahren nach Anspruch 3, **dadurch gekennzeichnet, daß** die Abluft durch einen Kühler der Industrieanlage geführt wird.

5. Verfahren nach Anspruch 3 oder 4, **dadurch gekennzeichnet, daß** die Abluft als Verbrennungsluft einem Brenner bzw. Ofen der Industrieanlage zugeführt wird.

6. Vorrichtung zum Abtöten von Keimen und anderen gesundheitsschädlichen Inhaltsstoffen in der Abluft von Müllagerstätten und/oder -sortieranlagen mit wenigstens einer Einrichtung zum Lagern und/oder Sortieren von Müll, die insbesondere nach dem Verfahren nach einem der Ansprüche 1 bis 5 betrieben wird,
**dadurch gekennzeichnet, daß** die Einrichtung (1, 3, 4, 23) über eine Verbindungsleitung (29, 30) mit einem Wärme abgebenden bzw. erzeugenden Teil (21) einer Industrieanlage (22) verbunden ist, in der die Abluft auf eine die Keime und anderen gesundheitsschädlichen Inhaltsstoffe abtötende Mindesttemperatur erhitzt wird.

7. Vorrichtung nach Anspruch 6, **dadurch gekennzeichnet, daß** der Teil (21) der Industrieanlage (22) ein Ofen ist.

8. Vorrichtung nach Anspruch 6, **dadurch gekennzeichnet, daß** der Teil (21) der Industrieanlage (22) ein Kühler ist.

9. Vorrichtung nach einem der Ansprüche 6 bis 8, **dadurch gekennzeichnet, daß** zwischen der Einrichtung (1, 3, 4, 23) und dem Teil (21) der Industrieanlage (22) ein die Abluft aus der Einrichtung (1, 3, 4, 23) absaugendes Gebläse (32, 33) angeordnet ist.

10. Vorrichtung nach Anspruch 8 oder 9, **dadurch gekennzeichnet, daß** der Kühler (21) wenigstens zwei Kühlkammern (25a, 25b, 25c) aufweist und daß die Abluft wahlweise einer oder mehreren Kühlkammern (25a, 25b, 25c) zuführbar ist.

11. Vorrichtung nach Anspruch 10, **dadurch gekennzeichnet, daß** die Abluft aus unterschiedlichen Abschnitten der Einrichtung (1, 3, 4, 23) wahlweise einer oder mehreren unterschiedlichen Kammern (25a, 25b, 25c) zuführbar ist.

12. Vorrichtung nach einem der Ansprüche 6 bis 11, **gekennzeichnet durch** einen Wärmetauscher (7), **durch** den einerseits die aus dem Teil (21) der Industrieanlage (22) austretende Luft und anderseits die Abluft aus der Einrichtung (1, 3) geführt ist.

13. Vorrichtung nach Anspruch 12, **dadurch gekennzeichnet, daß** in der Abluftleitung (29, 34) zwischen dem Wärmetauscher (7) und dem Teil (21) der Industrieanlage (22) eine Trockentrommel (4) für den Müll vorgesehen ist.

14. Vorrichtung nach Anspruch 13, **dadurch gekennzeichnet, daß** die Trockentrommel (4) zwei in achsialer Durchlaufrichtung des Mülls hintereinander angeordnete Abschnitte (4a, 4b) aufweist, die in entgegengesetzter Drehrichtung angetrieben sind.

15. Vorrichtung nach Anspruch 14, **dadurch gekennzeichnet, daß** die Abluftleitung (34) in dem in Durchlaufrichtung des Mülls nachgeordneten Abschnitt (4a) mündet.

16. Vorrichtung nach einem der Ansprüche 6 bis 15, **gekennzeichnet durch** eine Siebsichttrommel (12) und gegebenenfalls eine Nachsortiertrommel (17), die mit einer Verbindungsleitung (30) vorzugsweise über einen Staubabsichter (20) und mit dem Teil (21) der Industrieanlage (22) verbunden sind.

17. Vorrichtung nach einem der Ansprüche 8 bis 15, **gekennzeichnet durch** einen Kühler (21), der dem Brennofen einer Anlage (22) zur Zementklinkerherstellung zugeordnet ist.

## Claims

1. Method for destroying germs and other harmful components in the waste air from waste dumps and/or sorting plants, in which the waste air is heated, **characterised in that** the waste air is supplied to a part of an industrial plant which produces or discharges heat, and **in that** the waste air is heated to a minimum temperature which destroys the germs and other harmful components.

2. Method according to claim 1, **characterised in that** the waste air is supplied through a heat exchanger and a rotary drier for the waste of the industrial plant.

3. Method according to claim 2, **characterised in that** the heat exchanger is heated with the waste heat of the industrial plant.

4. Method according to claim 3, **characterised in that** the waste air is conducted through a cooler of the industrial plant.

5. Method according to claim 3 or 4, **characterised in that** the waste air is supplied as combustion air to a burner or furnace of the industrial plant.

6. Device for destroying germs and other harmful components in the waste air from waste dumps and/or sorting plants, having at least one appliance for storing and/or sorting waste, which is operated in particular according to the method according to one of the claims 1 to 5, **characterised in that** the appliance (1, 3, 4, 23) is connected via a connection line (29, 30) to a part (21) of an industrial plant (22) which discharges or produces heat, in which industrial plant the waste air is heated to a minimum temperature which destroys the germs and other harmful components.

7. Device according to claim 6, **characterised in that** the part (21) of the industrial plant (22) is a furnace.

8. Device according to claim 6, **characterised in that** the part (21) of the industrial plant (22) is a cooler.

9. Device according to one of the claims 6 to 8, **characterised in that**, between the appliance (1, 3, 4, 23) and the part (21) of the industrial plant (22), a blower (32, 33) is disposed, which suctions out the waste air from the appliance (1, 3, 4, 23).

10. Device according to claim 8 or 9, **characterised in that** the cooler (21) has at least two cooling chambers (25a, 25b, 25c) and **in that** the waste air can be supplied optionally to one or more cooling chambers (25a, 25b, 25c).

11. Device according to claim 10, **characterised in that** the waste air from different portions of the appliance (1, 3, 4, 23) can be supplied optionally to one or more different chambers (25a, 25b, 25c).

12. Device according to one of the claims 6 to 11, **characterised by** a heat exchanger (7) through which, on the one hand, the air discharging from the part (21) of the industrial plant (22) and, on the other hand, the waste air from the appliance (1, 3) is conducted.

13. Device according to claim 12, **characterised in that**, in the waste air line (29, 34) between the heat exchanger (7) and the part (21) of the industrial plant (22), a rotary drier (4) is provided for the waste.

14. Device according to claim 13, **characterised in that** the rotary drier (4) has two portions (4a, 4b) which are disposed one behind the other in the axial throughput direction of the waste and are driven in opposite directions of rotation.

15. Device according to claim 14, **characterised in that** the waste air line (34) discharges in the portion (4a) which is subsequently disposed in the throughput direction of the waste.

16. Device according to one of the claims 6 to 15, **characterised by** a screen classifying drum (12) and if necessary a post-classifying drum (17) which are connected to a connection line (30) preferably via a dust classifier (20) and to the part (21) of the industrial plant (22).

17. Device according to one of the claims 8 to 15, **characterised by** a cooler (21) which is assigned to the furnace of a plant (22) for cement clinker production.

## Revendications

1. Procédé pour tuer des germes et autres constituants nocifs pour la santé dans l'air d'échappement de décharges d'ordures et/ou d'installations de tri, dans lequel l'air évacué est réchauffé, **caractérisé en ce que** l'air d'échappement est amené à une partie d'une installation industrielle générant resp. émettant de la chaleur et **en ce que** l'air d'échappement est réchauffé jusqu'à une température minimale tuant les germes et autres constituants nocifs pour la santé.

2. Procédé selon la revendication 1, **caractérisé en ce que** l'air d'échappement est amené par un échangeur de chaleur et un tambour sec pour les ordures de l'installation industrielle.

3. Procédé selon la revendication 2, **caractérisé en ce que** l'échangeur de chaleur est réchauffé avec la chaleur dégagée de l'installation industrielle.

4. Procédé selon la revendication 3, **caractérisé en ce que** l'air d'échappement est guidé à travers un refroidisseur de l'installation industrielle.

5. Procédé selon la revendication 3 ou 4, **caractérisé en ce que** l'air d'échappement est amené comme air de combustion à un brûleur resp. un four de l'installation industrielle.

6. Dispositif pour tuer des germes et autres constituants nocifs pour la santé dans l'air d'échappement de décharges d'ordures et/ou d'installations de tri avec au moins un dispositif pour le stockage et/ou le tri d'ordures, qui est exploité en particulier selon le procédé conforme à l'une des revendications 1 à 5, **caractérisé en ce que** l'appareil (1, 3, 4, 23) est relié par une conduite de liaison (29, 30) à une partie (21), émettant resp. produisant de la chaleur, d'une installation industrielle (22), dans laquelle l'air d'échappement est réchauffé jusqu'à une température minimale tuant les germes et autres constituants nocifs pour la santé.

7. Dispositif selon la revendication 6, **caractérisé en ce que** la partie (21) de l'installation industrielle (22) est un four.

8. Dispositif selon la revendication 6, **caractérisé en ce que** la partie (21) de l'installation industrielle (22) est un refroidisseur.

9. Dispositif selon l'une quelconque des revendications 6 à 8, **caractérisé en ce qu'**une soufflerie (32, 33) évacuant par aspiration d'air d'échappement de l'appareil (1, 3, 4, 23) est disposée entre l'appareil (1, 3, 4, 23) et la partie (21) de l'installation industrielle (22).

10. Dispositif selon la revendication 8 ou 9, **caractérisé en ce que** le refroidisseur (21) présente au moins deux chambres de refroidissement (25a, 25b, 25c) et **en ce que** l'air d'échappement peut être amené au choix à une ou à plusieurs chambres de refroidissement (25a, 25b, 25c).

11. Dispositif selon la revendication 10, **caractérisé en ce que** l'air d'échappement provenant de différentes parties de l'appareil (1, 3, 4, 23) peut être amené au choix à l'une ou à plusieurs chambres (25a, 25b, 25c) différentes.

12. Dispositif selon l'une quelconque des revendications 6 à 11, **caractérisé par** un échangeur de chaleur (7), par lequel d'une part l'air sortant de la partie (21) de l'installation industrielle (22) et d'autre part l'air d'échappement sortant de l'appareil (1, 3) sont guidés.

13. Dispositif selon la revendication 12, **caractérisé en ce qu'**un tambour sec (4) pour les ordures est prévu dans la conduite d'air d'échappement (29, 34) entre l'échangeur de chaleur (7) et la partie (21) de l'installation industrielle (22).

14. Dispositif selon la revendication 13, **caractérisé en ce que** le tambour sec (4) présente deux parties (4a, 4b) disposées l'une derrière l'autre dans le sens de passage axial des ordures, lesquelles sont entraînées dans le sens de rotation opposé.

15. Dispositif selon la revendication 14, **caractérisé en ce que** la conduite d'air d'échappement (34) débouche dans la partie (4a) disposée en aval dans le sens de passage des ordures.

16. Dispositif selon l'une quelconque des revendications 6 à 15, **caractérisé par** le tambour de séparation cribleur (12) et éventuellement un tambour de tri complémentaire (17), qui sont reliés à une conduite de liaison (30) de préférence au moyen d'un séparateur de poussière (20) et à la partie (21) de l'installation industrielle (22).

17. Dispositif selon l'une quelconque des revendications 8 à 15, **caractérisé par** un refroidisseur (21), qui est attribué au four de cuisson d'une installation pour la fabrication de clinker.
